# EUROPEAN PATENT SPECIFICATION

(11) **EP 2 246 093 B1**
(45) Date of publication and mention of the grant of the patent: **22.04.2015**
(21) Application number: 10250853.8
(22) Date of filing: 28.04.2010
(51) Int. Cl.: A61N 1/372

(54) **Method and system for power management**
Power-Managementverfahren und -system
Procédé et système de gestion de l'alimentation

(30) Priority: 28.04.2009 US 431601
(43) Date of publication of application: 03.11.2010
(73) Proprietor: PACESETTER, INC., Sylmar, CA 91392-9221 (US)
(72) Inventor: Williamson, Richard, Santa Monica, CA 90403 (US); Dahlgren, Johan, 12061 Stockholm (SE); Lychou, Leif, 16345 Spanga (SE); Dianaty, Ali, Northridge, CA 91326 (US)
(74) Representative: Noble, Nicholas

(56) References cited:
- WO-A2-2008/070793
- US-A1- 2003 149 459
- US-A1- 2005 240 245
- US-A1- 2007 060 967

## Description

### BACKGROUND

### FIELD OF THE INVENTION

The present application relates generally to an implantable medical device and, more particularly, to a system and a method for using the system aimed at conserving the battery life of an implantable medical device.

### BACKGROUND OF THE INVENTION

Conserving the battery life of implantable medical devices (IMDs) such as pacemakers, defibrillators, and other neuron or muscle stimulator devices is very important for the purpose of extending the operating life of the IMD. A long or extended battery life extends the operating life of the IMD, thereby increasing the amount of time that device can remain within the patent and reducing the frequency of having to put a patient through unnecessary invasive operating procedures in order to replace the battery of the IMD. In a pacemaker, for example, intracardiac electrogram (IEGM) data are constantly being stored into the memory of the pacemaker. Using the IEGM data, doctors may perform an analysis on the patient and change the therapy programming as necessary to achieve a desired result. The pacemaker's microprocessor can also be configured to perform the IEGM data analysis and to make changes to the therapy programming automatically. However, such automation is limited by the complexity of the IEGM data analysis and the battery life of the IMD.

A complete IEGM data analysis may require a high level of micro-processing power and energy. Thus, it is advantageous to transmit the internally stored IEGM data and other telemetry data, such as operating status, battery status, other device status data or info, and/or other physiological data, to an external device where a complex analysis of the IEGM data and telemetry data may be performed without unnecessarily draining the battery of the IMD.

IMDs may transmit such telemetry data (including IEGM data) using various transmission methods such as by radio frequency (RF) means or induction means. Regardless of the mode of transmission, whenever the IMD transmits telemetry data to the external device, a high level of power is being consumed during the telemetry session (data transferring session). Thus, it is advantageous to limit the transmission of telemetry data where possible to preserve battery life.

US 2003/149459 details an implantable medical device including a radio-frequency (RF) telemetry circuit and a power connection module through which the RF telemetry circuit is connected to an energy source such as a battery. The power connection module connects power from the energy source to at least one portion of the RF telemetry circuit when a user initiates an RF telemetry session. After the RF telemetry session is completed, the power connection module shuts off the at least one portion of the RF telemetry circuit. Power-on examples include a wireless telemetry activation signal received by a low power radio receiver in the implantable device, a physical motion detected by an activity sensor in the implantable device, an activation of an inductive telemetry circuit in the implantable device, a magnetic field detected by a magnetic field detector in the implantable device, and/or a telemetry activation signal detected by a sensing circuit included in the implantable device. Power-off examples include a wireless germination signal received by the implantable device, a delay timeout following the RF telemetry session, and/or a signal received by an inductive telemetry circuit in the implantable device.

### SUMMARY

Aspects and features of the present disclosure are set out in the appended claims.

The following presents a simplified summary of one or more embodiments in order to provide a basic understanding of such embodiments. This summary is not an extensive overview of all contemplated embodiments, and is intended to neither identify key or critical elements of all embodiments nor delineate the scope of any or all embodiments. Its sole purpose is to present concepts of one or more embodiments in a simplified form as a prelude to the more detailed description that is presented later.

Routinely, physicians may want to extract telemetry data (e.g., IEGM data, other physiological data, operating data of the IMD, battery status, etc.) from an IMD in order to perform a full prognosis of the patient's condition and/or to alter the IMD therapy programming where necessary. Typically, while the IMD resides inside of a patient, it periodically or continuously collects and stores data into its memory. These stored data can then be extracted by a physician, other medical professional, medical technician or the like to an external device for further analysis. Extracting the stored data also allows the IMD to purge the stored data, thereby freeing up memory space for future data collection.

In addition to the stored telemetry data, physicians may also want to collect real-time telemetry data such as real-time IEGM data, other physiological data, and/or data relating to the IMD status while the patient is in the physician's office. This feature allows the physician to monitor and analyze the patient IEGM data and/or device data in real-time. However, transmitting telemetry data can consume a high level of power and shorten the life of the power source of the IMD if the telemetry session is not properly managed. Additionally, the operation of receiving data by the IMD also consumes battery power/current. Thus, it is advantageous to have built-in features to minimize the possibility that the IMD is transmitting, and the external device is receiving, telemetry data while it is not being monitored or used by the physician for an extended amount of time. It is also advantageous that the IMD be configured in a manner that conserves the life of the IMD power source when data receipt by the IMD is not necessary or desired.

In accordance with one or more embodiments and corresponding disclosure thereof, various aspects are described in connection with managing IMD communications, e.g., data transmission and/or data receipt, to save or conserve power. In particular, described herein are methods and systems for power and/or telemetry management for an IMD. The method, according to one embodiment of the present invention, may involve the steps of: receiving telemetry data transmitted from an IMD by an external device; determining a time lapse since receiving an input from a user at the external device while receiving the telemetry data; and sending an instruction from the external device and/or user to the IMD to terminate the telemetry data transmission to the external device when the time lapse has exceed a predetermined amount time. The instruction sent to the IMD can cause the IMD to terminate transmission of only selected telemetry data, in which case the telemetry connection between the external device and the IMD remains in place. Alternatively, the instruction sent to the IMD can cause the IMD to suspend or terminate transmission of all telemetry data, i.e., disconnecting the telemetry connection.

The method may also include the process of sending an instruction from the external device to the IMD to re-start the telemetry data transmission to the external device when an input from the user is detected after the predetermined amount of time has passed. The user interface of the external device can be a monitor, a keyboard, a touch screen, a mouse, or a combination of those devices, or other type of user interface known in the art. Additional types of user input devices useful in this regard include, and are not limited to, wireless slide advances, or wireless connections to the programmer or secondary device that can operate to capture the intent of the user to restart telemetry transmission.

In one embodiment, the instruction sent to the IMD may include an instruction to power down or turn off a transmitter in the IMD used for transmitting the telemetry data after a predetermined amount of time has lapsed since last user input. The predetermined amount may vary, and can be within the range of from about 1 to 15 minutes. In an example embodiment, the predetermined amount of time can be within the range of from about 2 to 10 minutes, with a preferred amount of time being from about 2 to 8 minutes.

In another embodiment, the method may periodically solicit the user's response to determine whether the user wants to continue receiving telemetry data at the external device. This can be for selected or all telemetry data. This may be done with a pop-up window or the like that appears on the external device, and that asks whether the user would like to continue receiving real-time telemetry data. The user may then be prompted to select 'yes' or 'no'. Depending on the selected answer, a signal may be sent to the IMD instructing it to stop transmitting real-time telemetry data, e.g., where the answer selected is "no". In one embodiment, if no answer is given within a predetermined amount of time, then a signal can be sent to the IMD instructing it to cease the telemetry transmitting session. This feature can be provided in addition to or as an alternative to the user manually selecting to terminate the telemetry transmission session.

In accordance with yet another embodiment of the invention, a system for conserving battery life of an IMD is provided. The system comprises: an external device comprising a communication module or the like, e.g., comprising a transmitter and receiver, configured to transmit signals to and receive telemetry data transmitted from an IMD; a timer to determine a time lapse since receiving an input from a user at the external device while receiving the telemetry data; and a controller configured to send an instruction signal from the external device to the IMD to terminate selected or all telemetry data transmission to the external device when the time lapse exceeds a predetermined amount time. In one embodiment, the signal provided by the external device may comprise an instruction to turn off or otherwise power down a transmitter in the IMD used for transmitting telemetry data.

The controller can be configured to send an instruction signal from the external device to the IMD to re-start or otherwise reinitiate the telemetry data transmission when an input is detected after the predetermined amount of time has passed. The instruction signal sent by the controller may include an instruction to power down a transmitter in the IMD used for transmitting telemetry data.

In one embodiment, the predetermined time lapse for terminating the transmission of telemetry data from the IMD may be within the range of from about 2 to 10 minutes, and preferably from about 2 to 8 minutes. Additionally, the controller can be configured to periodically solicit for a user response or user input to determine whether the user wants to continue receiving telemetry data at the external device. The controller can also be configured to send an instruction signal from the external device to the IMD to terminate selected or all telemetry data transmission to the external device based on the user response to a prompt at the external device.

In accordance with yet another embodiment of the invention, a computer program product is disclosed that comprises a computer useable medium having computer readable program code functions embedded in said medium for causing a computer to manage a telemetry transmission session of an IMD. The computer program product comprises: a first computer readable program code that causes the computer to receive telemetry data transmitted from an IMD at an external device; a second computer readable program code that causes the computer to determine a time lapse since receiving an input from a user at the external device while receiving the telemetry data; and a third computer readable program code that causes the computer to send an instruction signal from the external device to the IMD to terminate transmission of selected or all telemetry data to the external device when the time lapse exceeds a predetermined amount of time.

To the accomplishment of the foregoing and related ends, the one or more embodiments comprise the features hereinafter fully described and particularly pointed out in the claims. The following description and the annexed drawings set forth in detail certain illustrative aspects of the one or more embodiments. These aspects are indicative, however, of but a few of the various ways in which the principles of various embodiments may be employed and the described embodiments are intended to include all such aspects and their equivalents.

### BRIEF DESCRIPTION OF THE DRAWINGS

The present invention, in accordance with one or more various embodiments, is described in detail with reference to the following figures. The figures are provided for purposes of illustration only and merely depict typical or example embodiments of the invention. These drawings are provided to facilitate the reader's understanding of the invention and shall not be considered limiting of the breadth, scope, or applicability of the invention. It should be noted that for clarity and ease of illustration these figures are not necessarily made to scale.

FIG. 1 illustrates an exemplary environment with which the method and system of the present invention can be implemented according to one embodiment of the present invention;

FIG. 2 illustrates an exemplary system for power and telemetry management according to one embodiment of the present invention;

FIG. 3 illustrates an exemplary process flow chart for method of managing power and telemetry transmission of an IMD according to one embodiment of the present invention;

FIG. 4 illustrates an exemplary process flow chart for method of managing power and telemetry transmission of an IMD according to another embodiment of the present invention; and

FIG. 5 is a diagram illustrating an example computing system with which the power and telemetry managing method can be executed according to one embodiment of the present invention.

The figures are not intended to be exhaustive or to limit the invention to the precise form disclosed. It should be understood that the invention can be practiced with modification and alteration, and that the invention be limited only by the claims and the equivalents thereof.

### DETAILED DESCRIPTION

Various embodiments are now described with reference to the figures, wherein like reference numerals are used to refer to like elements throughout. In the following description, for purposes of explanation, numerous specific details are set forth in order to provide a thorough understanding of one or more embodiments. It may be evident, however, that such embodiment(s) can be practiced without these specific details. In other instances, well-known structures and devices are shown in block diagram form in order to facilitate describing one or more embodiments.

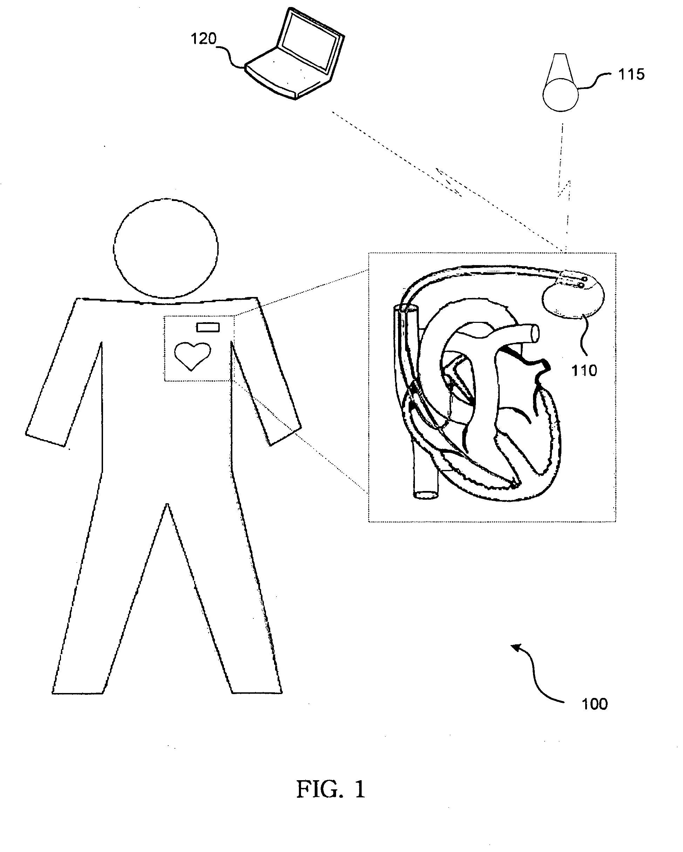

FIG. 1 illustrates an exemplary environment 100 with which the method and system of the current invention can be implemented. Referring to FIG. 1, the exemplary environment 100 is inside of the human body. As shown, an implantable medical device (IMD) 110 is surgically placed near the heart. The IMD 110 has leads that are operatively placed inside of one or more heart chambers. The IMD 110 can be a defibrillator or a pacemaker. However, it is to be understood that the method and system of the present invention is not intended to be limited to use with a defibrillator or pacemaker. Rather, the invention can be applied in various types of IMDs such as tissue/muscle stimulators, neuron stimulators, or any other type of medical device having telemetry that depends on an internal power source (i.e., a power source such as a battery or the like that is positioned inside of the patient's body).

The IMD 110 has a wireless telemetry capability that allows it to communicate with an external device, i.e., a device that is positioned outside of the human body, such as a communication wand 115 or a computer 120, or the like. The IMD 110 can be configured to communicate with the communication wand 115 or external device using various communication methods such as magnetic induction, acoustic communication, near field communication, far field communication, and the like.

In one embodiment, the IMD 110 is configured to communicate with an external programmer such as computer 120 or the like using a RF communication link. In this embodiment, both the IMD 110 and the external device such as the computer 120 have an RF transceiver and/or receiver that are capable of transmitting and receiving data using various RF communication methods. In this way, the IMD 110 may transfer telemetry data (e.g. IEGM data, physiological data, operating data, battery status, and the lead voltage and impedance data) to the external device for desired analysis. Based on the analysis, the therapy programming of the IMD can be changed using the same RF communication method.

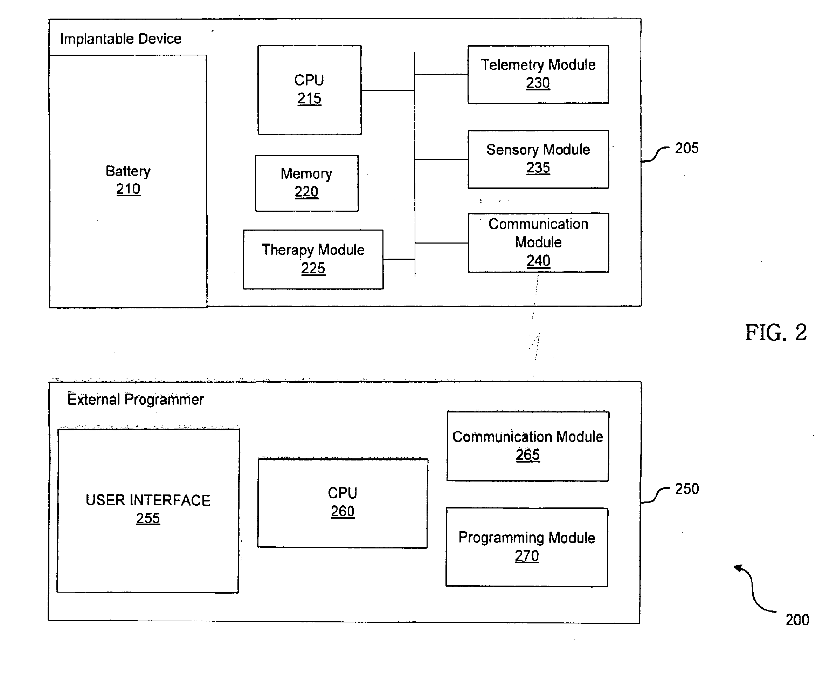

FIG. 2 illustrates an example system 200 having power and telemetry management features according to one embodiment of the present invention. The system 200 includes an IMD 205 and an external programmer 250. The IMD 205 may be a pacemaker or a defibrillator. The external programmer 250 may be a handheld computer, a laptop, or a workstation. As shown in FIG. 2, the IMD 205 may include a battery 210, a central processing unit (CPU) 215, a memory 220, a therapy module 225, a telemetry module 230, a sensory module 235, and a communication module 240. The external programmer 250 may include a user interface 255, a CPU 260, a communication module 265, and a programming module 270. The user interface 255 can be a keyboard, a touch screen, a mouse and a monitor, or a combination thereof. The transceiver 240 of the IMD 205 and the transceiver 265 of the external programmer 250 are configured to communicate with each other using various RF communication methods such as frequency modulation, amplitude modulation, or other RF communication method.

In one embodiment, the communication module 240 is a RF transceiver configured to transmit telemetry data collected by telemetry module 230 to an external device such as the external programmer 250. The telemetry module 230 may collect various data such as the operating status of IMD 205, status of the power source 210, e.g., a battery or the like, the number of times therapy was applied, IEGM data, the lead impedance and voltages, and other physiological data. The telemetry data collected by the telemetry module 230 can be stored in the memory 220. The sensory module 235 may include various physiological measurement instruments for collecting heart rate data, atrial intracardiac electrogram data, and ventricular intracardiac electrogram data, and any other suitable physiological data collected by the telemetry module 230. Based on the information measured by the sensory module 235, the therapy module 225 may execute various therapy programs on the patient such as synchronization pacing, bradycardia pacing, anti-tachycardia pacing, or other suitable type of pacing program.

The IMD 205 can be placed into a telemetry transmit mode in several ways. The IMD 205 may start transmitting telemetry data when it receives an information request signal from the external programmer 250, or when it detects a change in the electromagnetic field caused by placement of a communication wand (shown in FIG. 1 as 115) or the like being placed nearby the IMD. The IMD can also be configured to initiate transmission of telemetry data when it detects a change in the electric or magnetic fields independent of a communication wand that corresponds to a signal for communication to be initiated. Additionally, the IMD can be configured to restart radio frequency telemetry with radio frequency commands.

The IMD 205 may also include one or more accelerometers (not shown) to detect sharp movements caused by taps on patient's skin where the IMD 205 is approximately located. The tappings can be a physical cue telling the IMD 205 to start transmitting telemetry data. In the system 200, to start a telemetry session, the external programmer 250 would send an information request signal to IMD 205.

The IMD can be configured to initiate telemetry transmission, or enter a telemetry transmit mode, when the IMD detects a medical event, e.g., detecting a component failure which may have adverse effects on IMD therapy, or when it detects a hazardous arrhythmia, e.g., in the case where the IMD is a pacemaker or implantable defibrillator. In such embodiment, the IMD can spontaneously start transmitting without any request from the external device.

Depending on the type of information request signal received, the IMD 205 may transmit telemetry data as follows: partially transmit the telemetry data stored in memory 220, for example, the information request signal may only ask for data between certain dates; transmit all of the telemetry data stored in the memory 220; and/or transmit real-time IEGM data. The telemetry data transmitted can be selected telemetry data, where such selected data could be, but is not limited to, IEGM data. In one embodiment, the IMD erases the telemetry data from memory 220 after the telemetry data are transmitted to the external device 250. In this way, the memory 220 can have sufficient space for future storage needs.

The CPU 215 or a controller of the IMD 205 can be configured to monitor the status of the telemetry data session (transmitting telemetry data to an external device). As previously mentioned, when the IMD 205 is transmitting telemetry data it consumes a high level of power that reduces the life of the battery. Thus, it is advantageous to minimize the length of the telemetry data transmission session. In one embodiment, the CPU 215 is configured to power down the transceiver 240 when it is not in use and when a telemetry data session is completed.

The CPU 215 or a controller of the IMD 205 can also be configured to monitor for a periodic feedback signal from the external device whenever the IMD 205 is transmitting real-time IEGM data to an external device. The CPU 215 may continue or terminate the telemetry data transmission session based on the feedback signal. The feedback signal may instruct the IMD 205 to continue or discontinue transmitting real-time telemetry data (e.g., real-time IEGM data). While the feedback signal has been described as a signal that is generated and sent by the external device to the IMD, it is to be understood that the IMD can also be configured to terminate transmission in the event that it does not receive an instruction from the external device, i.e., in the absence of the external device sending an instruction to continue sending data.

In one embodiment, the CPU 215 is configured to terminate the telemetry session and to power down the transceiver 240 when a predetermined amount of time has lapsed since a feedback signal has been received from the external programmer 250. Alternatively, the CPU can be configured to terminate or suspend the transmission of selected telemetry data from the IMD, in which case the telemetry connection remains in place for the possibly transmission of other nonselected telemetry data. The predetermined amount of time for terminating a telemetry session can vary depending on a number of variables such as the storage capacity of the particular power source powering the IMD, but in an example embodiment can range between about 1 to 15 minutes. In an example embodiment, the predetermined amount of time is about 2 to 8 minutes. In this way, if the external programmer is not being monitored by a physician or if the patient somehow walked out of range, then the IMD 205 may turn off the telemetry session on its own to save battery life.

The external programmer 250 is also configured with similar telemetry session terminating features. While receiving real-time telemetry data from the IMD 205, the programmer 250 may periodically require the physician to input a command using the user interface 255. For example, programmer 250 may be configured to inquire every 5 minutes on whether the physician wishes to continue receiving real-time telemetry data. The physician could be prompted to enter or select 'yes' or 'no' using the user interface 255. If 'no' is selected, the programmer 250 may send an instruction to the IMD 205 commanding it to terminate or suspend the telemetry session. If 'yes' is selected, the programmer 250 may send an instruction to the IMD 205 commanding it to continue sending real-time telemetry data. In one embodiment, if the external programmer 250 has not detected any type of user input at the user interface 255 for a predetermined amount of time, then the programmer 250 may send an instruction to the IMD 205 commanding it to terminate the telemetry session and power down the transceiver 240. The predetermined amount of time may range from about 2 to 10 minutes, and preferably being about 2 to 8 minutes.

The external programmer 250 may also command the IMD to restart the telemetry session once it has detected a user input at the user interface. For example, the IMD 205 may be transmitting real-time telemetry data for the last 5 minutes but the external programmer 250 has not received any input from the user. If the predetermined time for terminating the telemetry data transmission session is 5 minutes, then an instruction is sent to the IMD 205 commanding it to stop the telemetry session. However, the external programmer 250 may re-start the telemetry session once it detects that the physician is inputting information again at the user interface 240.

In one embodiment, the physician may start or terminate a telemetry session using the user interface 255. For example, the physician may choose to start a telemetry session by selecting a "start telemetry" button displayed on the user interface 255. Once the button is selected, the programmer 250 sends an instruction to the IMD 205 commanding it to start a telemetry session with the external programmer 250.

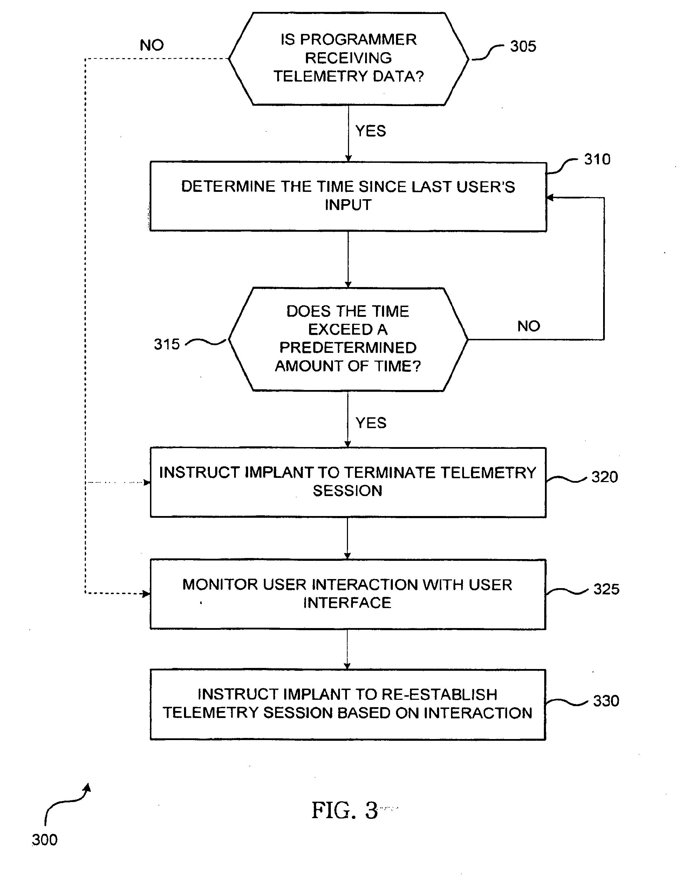

FIG. 3 illustrates an operational flow diagram 300 for managing the power and telemetry of IMD 205 according to one example embodiment of the present invention. The operational flow 300 starts at step 305 where it is determined whether the external programmer is receiving telemetry data. If 'yes', the process proceeds to step 310 where the time lapse since the last user's input is determined. This time lapse is used to determine how long the external programmer has been receiving telemetry data while not receiving any user input.

In step 315, the calculated time lapse is compared with a predetermined time to determine whether the calculated time lapse exceeds the predetermined time. If 'yes', the external programmer sends a command to the IMD to terminate the telemetry session in step 320. Again, this command can operate to either disconnect the telemetry connection entirely, or to suspend the transmission of certain selected telemetry data while leaving the telemetry connection intact for possible transmission of other telemetry data. In this way, the IMD is prevented from sending telemetry data to the external device while nobody is monitoring the data. In one embodiment, in addition to terminating the telemetry session, the IMD can also power down the transmitter to save additional energy.

In step 325, the user interface is monitored for any user input. For example, if the physician has returned and is inputting commands into the external programmer, it can instruct the IMD to resume the telemetry session, as shown in step 330.

If the answer in step 305 is 'no', then the external programmer may optionally send a command to the IMD to terminate any telemetry session currently in progress at step 320. The external programmer may additionally instruct the IMD to power down the RF transmitter used for transmitting the telemetry data. Further, the external programmer can monitor for any interaction with the user interface.

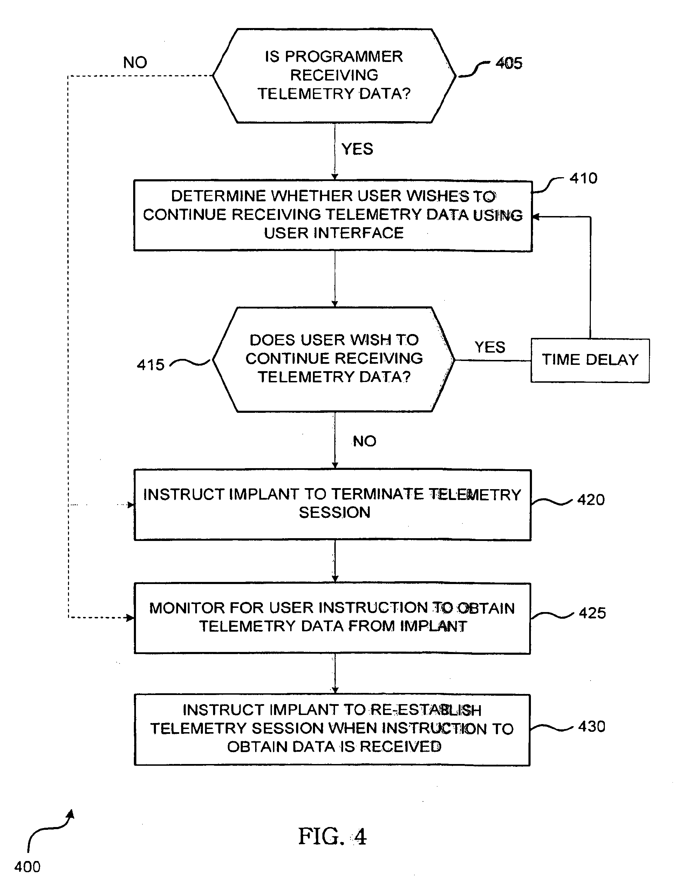

FIG. 4 illustrates an operational flow diagram 400 for managing power and telemetry of IMD 205 according to another example embodiment of the present invention. Referring now to FIG. 4, operational flow diagram starts at step 405 where it is determined whether the programmer is currently receiving telemetry data. If 'yes', the process proceeds to steps 410 and 415 where the user is periodically asked via a user interface, after a predetermined time delay as indicated between 415 and 410, to determine whether they want to continue receiving telemetry data at the external device. This may be done using a pop-up window on the user interface that asks whether the user would like to continue receiving telemetry data. The user is then required to select 'yes' or 'no'. Depending on the selected answer, an instruction may be sent to the IMD instructing it to stop transmitting telemetry data. In one embodiment, if a 'no' answer is given, then an instruction will be sent to the IMD instructing it to cease the telemetry session as shown in step 420. As noted above, this instruction can operate to either disconnect the telemetry connection entirely, or to suspend the transmission of certain selected telemetry data while leaving the telemetry connection intact for possible transmission of other telemetry data

Once the telemetry session is terminated or suspended, the external programmer may give the user a choice to restart the telemetry session through the user interface of the external device. In step 425, the external device is configured to monitor for input from the user that indicates the user's desire to restart the telemetry session. If 'yes', the external programmer sends an instruction to the IMD, commanding it to restart the telemetry session in step 430.

The system 200, and the example embodiments described in operational flow diagrams 300 and 400, generally operate to place the IMD into a suspended communication mode, to suspend data transmission and/or data receipt, either controlled by a timer or on user request to conserve the life of the power source. The system may employ a RF-sniffing or RF detection technique to conserve power. Generally, it takes power for a transceiver to sniff for RF communication attempts by other transceivers. Thus, it is beneficial to control the time intervals between such sniffs to conserve power. For times directly after a telemetry data transmission session has ended, the sniff interval can be set to a short amount of time to anticipate a situation where communication might need to be quickly reestablished. If communication is not reestablished after a predetermined amount of time, then the sniff interval can be configured to go for a longer period of time or time interval to save power. Additionally or alternatively, the frequency of the RF sniffing channel can be set in a manner that is power sensitive and indicative of the use conditions. In an example embodiment, the IMD is configured such that, when placed in a suspended mode, it consumes less and less current the longer the communication pause persists, i.e., the current consumption decreases as the communication interval increases.

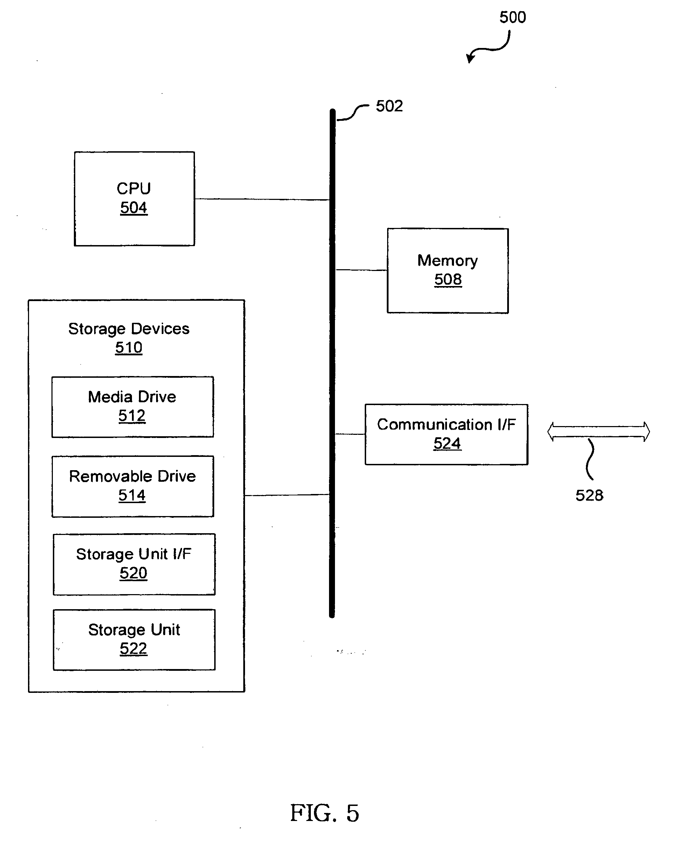

Where components or modules of the invention are implemented in whole or in part using software, in one embodiment, these software elements can be implemented to operate with a computing or processing module capable of carrying out the functionality described with respect thereto. One such example embodiment computing module is shown in FIG. 5. Various embodiments are described in terms of this example-computing module 500. After reading this description, it will become apparent to a person skilled in the relevant art how to implement the invention using other computing modules or architectures.

Referring now to FIG. 5, the computing module 500 may represent, for example, computing or processing capabilities found within desktop, laptop and notebook computers; hand-held computing devices (PDA's, smart phones, cell phones, palmtops, etc.); mainframes, supercomputers, workstations or servers; or any other type of special-purpose or general-purpose computing devices as may be desirable or appropriate for a given application or environment. The computing module 500 might also represent computing capabilities embedded within or otherwise available to a given device. For example, a computing module might be found in other electronic devices such as, for example, digital cameras, navigation systems, cellular telephones, portable computing devices, modems, routers, WAPs, and other electronic devices that might include some form of processing capability.

The computing module 500 might include, for example, one or more processors or processing devices, such as a processor or CPU 504. The processor 504 might be implemented using a general-purpose or special-purpose processing engine such as, for example, a microprocessor, controller, or other control logic. In the example illustrated in FIG. 5, the processor 504 is connected to a bus 502 or other communication medium to facilitate interaction with other components of the computing module 500.

The computing module 500 might also include one or more memory modules, referred to as main memory 508. For example, preferably random access memory (RAM) or other dynamic memory, might be used for storing information and instructions to be executed by the processor 504. The main memory 508 might also be used for storing temporary variables or other intermediate information during execution of instructions to be executed by the processor 504. The computing module 500 might likewise include a read only memory ("ROM") or other static storage device coupled to the bus 502 for storing static information and instructions for the processor 504.

The computing module 500 might also include one or more various forms of an information storage mechanism 510, which might include, for example, a media drive 512 and a storage unit interface 520. The media drive 512 might include a drive or other mechanism to support the fixed or removable storage media 514. For example, a hard disk drive, a floppy disk drive, a magnetic tape drive, an optical disk drive, a CD or DVD drive (R or RW), or other removable or fixed media drive. Accordingly, the storage media 514, might include, for example, a hard disk, a floppy disk, magnetic tape, cartridge, optical disk, a CD or DVD, or other fixed or removable medium that is read by, written to or accessed by the media drive 512. As these examples illustrate, the storage media 514 can include a computer usable storage medium having stored therein particular computer software or data.

In alternative embodiments, the information storage mechanism 510 might include other similar instrumentalities for allowing computer programs or other instructions or data to be loaded into the computing module 500. Such instrumentalities might include, for example, a fixed or removable storage unit 522 and an interface 520. Examples of such storage units 522 and interfaces 520 can include a program cartridge and cartridge interface, a removable memory (for example, a flash memory or other removable memory module) and memory slot, a PCMCIA slot and card, and other fixed or removable storage units 522 and interfaces 520 that allow software and data to be transferred from the storage unit 522 to the computing module 500.

The computing module 500 might also include a communications interface 524. The communications interface 524 might be used to allow software and data to be transferred between the computing module 500 and external devices. Examples of communications interface 524 might include a modem or softmodem, a network interface (such as an Ethernet, network interface card, WiMedia, 802.XX or other interface), a communications port (such as for example, a USB port, IR port, RS232 port Bluetooth interface, or other port), or other communications interface. Software and data transferred via communications the interface 524 might typically be carried on signals, which can be electronic, electromagnetic, optical or other signals capable of being exchanged by a given communications the interface 524. These signals might be provided to the communications interface 524 via a channel 528. This channel 528 might carry signals and might be implemented using a wired or wireless medium. Some examples of a channel might include a phone line, a cellular link, an RF link, an optical link, a network interface, a local or wide area network, and other wired or wireless communications channels.

In this document, the terms "computer program medium" and "computer usable medium" are used to generally refer to media such as, for example, the memory 508, the storage unit 520, the media 514, and signals on the channel 528. These and other various forms of computer program media or computer usable media may be involved in carrying one or more sequences of one or more instructions to a processing device for execution. Such instructions embodied on the medium, are generally referred to as "computer program code" or a "computer program product" (which may be grouped in the form of computer programs or other groupings). When executed, such instructions might enable the computing module 500 to perform features or functions of the present invention as discussed herein.

Unless defined otherwise, all technical and scientific terms used herein have the same meaning as is commonly understood by one of ordinary skill in the art to which this invention belongs. If a definition set forth in this section is contrary to or otherwise inconsistent with a definition set forth in applications, published applications and other publications that are herein incorporated by reference, the definition set forth in this section prevails over the definition that is incorporated herein by reference.

The term tool can be used to refer to any apparatus configured to perform a recited function. For example, tools can include a collection of one or more modules and can also be comprised of hardware, software or a combination thereof. Thus, for example, a tool can be a collection of one or more software modules, hardware modules, software/hardware modules or any combination or permutation thereof. As another example, a tool can be a computing device or other appliance on which software runs or in which hardware is implemented.

As used herein, the term module might describe a given unit of functionality that can be performed in accordance with one or more embodiments of the present invention. As used herein, a module might be implemented utilizing any form of hardware, software, or a combination thereof. For example, one or more processors, controllers, ASICs, PLAs, logical components, software routines or other mechanisms might be implemented to make up a module. In implementation, the various modules described herein might be implemented as discrete modules or the functions and features described can be shared in part or in total among one or more modules. In other words, as would be apparent to one of ordinary skill in the art after reading this description, the various features and functionality described herein may be implemented in any given application and can be implemented in one or more separate or shared modules in various combinations and permutations. Even though various features or elements of functionality may be individually described or claimed as separate modules, one of ordinary skill in the art will understand that these features and functionality can be shared among one or more common software and hardware elements, and such description shall not require or imply that separate hardware or software components are used to implement such features or functionality.

Additionally, the various embodiments set forth herein are described in terms of exemplary block diagrams, flow charts and other illustrations. As will become apparent to one of ordinary skill in the art after reading this document, the illustrated embodiments and their various alternatives can be implemented without confinement to the illustrated examples. For example, block diagrams and their accompanying description should not be construed as mandating a particular architecture or configuration.

The previous description of the disclosed examples is provided to enable any person skilled in the art to make or use the present invention. Various modifications to these examples will be readily apparent to those skilled in the art, and the generic principles defined herein may be applied to other examples without departing from the scope of the invention. Thus, the present invention is not intended to be limited to the examples shown herein but is to be accorded the widest scope consistent with the claims.

## Claims

1. A method for power management comprising the steps of:
receiving telemetry data in the form of real-time intracardiac electrogram, IEGM, data transmitted from an implantable medical device (205) to an external device (250);
determining (310) a time lapse since receiving an input from a user at the external device (250) while receiving the real-time IEGM data; and
sending (320) an instruction from the external device (250) to the implantable medical device (205) to terminate the transmission of real-time IEGM data to the external device (250) when the time lapse exceeds a predetermined amount time.

2. The method as recited in claim 1 wherein after the step of sending, transmission of selected real-time IEGM data is terminated and a telemetry connection between the implantable medical device (205) and the external device (250) remains in place or wherein after the step of sending, a telemetry connection between the implantable medical device (205) and the external device (250) is suspended.

3. The method as recited in claim 1 further comprising the steps of sending (330) an instruction from the external device (250) to the implantable medical device (205) to re-start the transmission of real-time IEGM data to the external device (250) when an input from the user is detected after the predetermined amount of time has passed.

4. The method as recited in claim 1 wherein the user input is received at a user interface of the external device (250).

5. The method as recited in claim 1 wherein the instruction comprises an instruction to power down a transmitter in the implantable medical device (205) used for transmitting the real-time IEGM data.

6. The method as recited in claim 1 wherein the predetermined amount of time is between about 2 to 8 minutes.

7. The method as recited in claim 1 further comprising the step of soliciting for a user response to determine whether the user wants to continue receiving real-time IEGM data at the external device (250) or further comprising the step of sending an instruction from the external device (250) to the implantable medical device (205) to terminate the real-time IEGM data transmission to the external device (250) based on an input from the user.

8. The method as recited in claim 7 wherein the method of soliciting is performed using a user interface that is connected with the external device (250), wherein the method of soliciting is preferably performed at periodic intervals.

9. The method as recited in claim 1 wherein after transmission of the real-time IEGM data from the implantable medical device (205) has been terminated, the further step of the implantable medical device (205) seeking for a signal to initiate transmission.

10. The method as recited in claim 9 wherein the step of seeking is performed at an interval of time that varies depending on the time since transmission termination, wherein the interval of time preferably increases in duration if no signal to initiate transmission is sensed.

11. A system for managing the battery life of an implantable medical device (205) comprising:
a transceiver configured to receive telemetry data in the form of real-time intracardiac electrogram, IEGM, data transmitted from the implantable medical device (205) to an external device (250);
a timer to determine a time lapse since receiving an input from a user at the external device (250) while receiving the real-time IEGM data; and
a controller configured to send an instruction from the external device (250) to the implantable medical device (205) to terminate transmission of the real-time IEGM data to the external device (250) when the time lapse exceeds a predetermined amount time.

12. The system as recited in claim 11 wherein after receiving the instruction the implantable medical device (205) terminates transmission of selected real-time IEGM data and a telemetry connection between the implantable medical device (205) and the external device (250) remains in place or wherein after receiving the instruction the implantable medical device (205) terminates transmission of all real-time IEGM data and a telemetry connection between the implantable medical device (205) and the external device (250) is suspended.

13. The system as recited in claim 11 wherein the controller is further configured to send an instruction from the external device (250) to the implantable medical device (205) to re-start transmission of real-time IEGM data to the external device (250) when an input from the user is detected after the predetermined amount of time has passed.

14. The system of recited in claim 11 wherein the instruction comprises an instruction to power down a transmitter in the implantable medical device (205) used for transmitting real-time IEGM data.

15. The system as recited in claim 11 wherein the controller is further configured to periodically solicit for a user response to determine whether the user wants to receive real-time IEGM data at the external device (250).

## Patentansprüche

1. Verfahren zum Energiemanagement, umfassend die folgenden Schritte:
Empfangen von Telemetriedaten in der Form von Echtzeitdaten eines intrakardialen Elektrogramms, IEKG, die von einem implantierbaren Medizinprodukt (205) an eine externe Vorrichtung (250) übertragen werden;
Bestimmen (310) eines Zeitablaufs seit dem Empfangen einer Eingabe von einem Benutzer an der externen Vorrichtung (250) während des Empfangens der Echtzeit-IEKG-Daten; und
Senden (320) einer Anweisung von der externen Vorrichtung (250) an das implantierbare Medizinprodukt (205), die Übertragung von Echtzeit-IEKG-Daten an die externe Vorrichtung (250) zu beenden, wenn der Zeitablauf eine vorbestimmte Zeitspanne überschreitet.

2. Verfahren nach Anspruch 1, wobei nach dem Schritt des Sendens die Übertragung von ausgewählten Echtzeit-IEKG-Daten beendet wird und eine Telemetrieverbindung zwischen dem implantierbaren Medizinprodukt (205) und der externen Vorrichtung (250) bestehen bleibt oder wobei nach dem Schritt des Sendens eine Telemetrieverbindung zwischen dem implantierbaren Medizinprodukt (205) und der externen Vorrichtung (250) unterbrochen wird.

3. Verfahren nach Anspruch 1, des Weiteren umfassend die Schritte des Sendens (330) einer Anweisung von der externen Vorrichtung (250) an das implantierbare Medizinprodukt (205), die Übertragung von Echtzeit-IEKG-Daten an die externe Vorrichtung (250) erneut zu starten, wenn eine Eingabe vom Benutzer erfasst wird, nachdem die vorbestimmte Zeitspanne verstrichen ist.

4. Verfahren nach Anspruch 1, wobei die Benutzereingabe an einer Benutzerschnittstelle der externen Vorrichtung (250) empfangen wird.

5. Verfahren nach Anspruch 1, wobei die Anweisung eine Anweisung umfasst, einen Sender im implantierbaren Medizinprodukt (205) abzuschalten, der für das Übertragen der Echtzeit-IEKG-Daten verwendet wird.

6. Verfahren nach Anspruch 1, wobei die vorbestimmte Zeitspanne zwischen ungefähr 2 bis 8 Minuten beträgt.

7. Verfahren nach Anspruch 1, des Weiteren umfassend den Schritt des Anforderns einer Benutzerantwort, um zu bestimmen, ob der Benutzer das Empfangen von Echtzeit-IEKG-Daten an der externen Vorrichtung (250) fortsetzen möchte, oder des Weiteren umfassend den Schritt des Sendens einer Anweisung von der externen Vorrichtung (250) an das implantierbare Medizinprodukt (205), die Übertragung von Echtzeit-IEKG-Daten an die externe Vorrichtung (250) auf der Grundlage einer Eingabe vom Benutzer zu beenden.

8. Verfahren nach Anspruch 7, wobei das Verfahren des Anforderns mittels einer Benutzerschnittstelle durchgeführt wird, die mit der externen Vorrichtung (250) verbunden ist, wobei das Verfahren des Anforderns vorzugsweise in periodischen Intervallen durchgeführt wird.

9. Verfahren nach Anspruch 1, wobei nach dem Beenden der Übertragung der Echtzeit-IEKG-Daten vom implantierbaren Medizinprodukt (205) der weitere Schritt des implantierbaren Medizinprodukts (205) das Suchen nach einem Signal zum Einleiten der Übertragung ist.

10. Verfahren nach Anspruch 9, wobei der Schritt des Suchens in einem Zeitintervall durchgeführt wird, das je nach der Zeit seit der Beendigung der Übertragung variiert, wobei sich die Dauer des Zeitintervalls vorzugsweise verlängert, wenn kein Signal zum Einleiten der Übertragung erfasst wird.

11. System zum Verwalten der Lebensdauer einer Batterie eines implantierbaren Medizinprodukts (205), umfassend:
einen Sender/Empfänger, der konfiguriert ist, Telemetriedaten in der Form von Echtzeitdaten eines intrakardialen Elektrogramms, IEKG, zu empfangen, die vom implantierbaren Medizinprodukt (205) an eine externe Vorrichtung (250) übertragen werden;
einen Timer zum Bestimmen eines Zeitablaufs seit dem Empfangen einer Eingabe von einem Benutzer an der externen Vorrichtung (250) während des Empfangens der Echtzeit-IEKG-Daten; und
eine Steuerung, die konfiguriert ist, eine Anweisung von der externen Vorrichtung (250) an das implantierbare Medizinprodukt (205) zu senden, die Übertragung der Echtzeit-IEKG-Daten an die externe Vorrichtung (250) zu beenden, wenn der Zeitablauf eine vorbestimmte Zeitspanne überschreitet.

12. System nach Anspruch 11, wobei nach dem Empfangen der Anweisung das implantierbare Medizinprodukt (205) die Übertragung von ausgewählten Echtzeit-IEKG-Daten beendet und eine Telemetrieverbindung zwischen dem implantierbaren Medizinprodukt (205) und der externen Vorrichtung (250) bestehen bleibt oder wobei nach dem Empfangen der Anweisung das implantierbare Medizinprodukt (205) die Übertragung aller Echtzeit-IEKG-Daten beendet und eine Telemetrieverbindung zwischen dem implantierbaren Medizinprodukt (205) und der externen Vorrichtung (250) unterbrochen wird.

13. System nach Anspruch 11, wobei die Steuerung des Weiteren konfiguriert ist, eine Anweisung von der externen Vorrichtung (250) an das implantierbare Medizinprodukt (205) zu senden, die Übertragung von Echtzeit-IEKG-Daten an die externe Vorrichtung (250) erneut zu starten, wenn eine Eingabe vom Benutzer erfasst wird, nachdem die vorbestimmte Zeitspanne verstrichen ist.

14. System nach Anspruch 11, wobei die Anweisung eine Anweisung umfasst, einen Sender im implantierbaren Medizinprodukt (205) abzuschalten, der für das Übertragen von Echtzeit-IEKG-Daten verwendet wird.

15. System nach Anspruch 11, wobei die Steuerung des Weiteren konfiguriert ist, periodisch eine Benutzerantwort anzufordern, um zu bestimmen, ob der Benutzer Echtzeit-IEKG-Daten an der externen Vorrichtung (250) empfangen möchte.

## Revendications

1. Un procédé de gestion de l'énergie comprenant les étapes suivantes :
recevoir des données de télémétrie sous la forme d'un électrogramme intracardiaque (IEGM) en temps réel et transmises d'un dispositif médical implantable (205) à un appareil externe (250) ;
déterminer (310) le délai écoulé depuis la réception d'une saisie de la part d'un utilisateur sur l'appareil externe (250) tout en recevant les données d'IEGM en temps réel ; et
envoyer (320) depuis l'appareil externe (250) une instruction indiquant au dispositif médical implantable (205) d'interrompre la transmission des données d'IEGM en temps réel audit appareil externe (250) lorsque le délai écoulé dépasse une durée prédéterminée.

2. Le procédé décrit à la revendication 1, dans le cadre duquel, après l'étape de l'envoi de l'instruction, la transmission des données d'IEGM en temps réel sélectionnées est interrompue et une connexion de télémétrie entre le dispositif médical implantable (205) et l'appareil externe (250) reste établie ou dans le cadre duquel, après l'étape de l'envoi de l'instruction, une connexion de télémétrie entre le dispositif médical implantable (205) et l'appareil externe (250) est suspendue.

3. Le procédé décrit à la revendication 1, comprenant en outre l'étape suivante :
envoyer (320) depuis l'appareil externe (250) une instruction indiquant au dispositif médical implantable (205) de redémarrer la transmission des données d'IEGM en temps réel audit appareil externe (250) lorsqu'une saisie de la part de l'utilisateur est détectée après l'écoulement de la durée prédéterminée.

4. Le procédé décrit à la revendication 1, dans le cadre duquel la saisie de l'utilisateur est reçue via une interface de l'appareil externe (250).

5. Le procédé décrit à la revendication 1, dans le cadre duquel l'instruction comprend une instruction indiquant d'éteindre un émetteur situé dans le dispositif médical implantable (205) et utilisé pour transmettre les données d'IEGM en temps réel.

6. Le procédé décrit à la revendication 1, dans le cadre duquel la durée prédéterminée est comprise entre 2 et 8 minutes environ.

7. Le procédé décrit à la revendication 1, comprenant en outre l'étape suivante :
demander à l'utilisateur s'il souhaite continuer à recevoir les données d'IEGM en temps réel sur l'appareil externe (250) ou passer à l'étape consistant à envoyer (320) depuis l'appareil externe (250) une instruction indiquant au dispositif médical implantable (205) d'interrompre la transmission des données d'IEGM en temps réel audit appareil externe (250) d'après une saisie de l'utilisateur.

8. Le procédé décrit à la revendication 7, dans le cadre duquel la demande s'effectue via une interface connectée à l'appareil externe (250) et de préférence à intervalle régulier.

9. Le procédé décrit à la revendication 1, dans le cadre duquel, après l'interruption de la transmission des données d'IEGM en temps réel depuis le dispositif médical implantable (205), ce dernier recherche un signal de redémarrage de la transmission.

10. Le procédé décrit à la revendication 9, dans le cadre duquel l'étape de recherche du signal s'effectue à un intervalle de temps qui varie selon le temps écoulé depuis l'interruption de la transmission et, de préférence, augmente progressivement lorsqu'aucun signal de redémarrage de la transmission n'est détecté.

11. Un procédé de gestion de l'autonomie d'un dispositif médical implantable (205) comprenant les éléments suivants :
un émetteur/récepteur conçu pour recevoir des données de télémétrie sous la forme d'un électrogramme intracardiaque (IEGM) en temps réel et transmises dudit dispositif médical implantable (205) à un appareil externe (250) ;
un minuteur servant à déterminer le délai écoulé depuis la réception d'une saisie de la part d'un utilisateur sur l'appareil externe (250) tout en recevant les données d'IEGM en temps réel ; et
un contrôleur conçu pour envoyer depuis l'appareil externe (250) une instruction indiquant au dispositif médical implantable (205) d'interrompre la transmission des données d'IEGM en temps réel audit appareil externe (250) lorsque le délai écoulé dépasse une durée prédéterminée.

12. Le système décrit à la revendication 11, dans le cadre duquel, après avoir reçu l'instruction, le dispositif médical implantable (205) interrompt la transmission des données d'IEGM en temps réel sélectionnées tandis qu'une connexion de télémétrie entre le dispositif médical implantable (205) et l'appareil externe (250) reste établie ou dans le cadre duquel, après avoir reçu l'instruction, le dispositif médical implantable (205) interrompt la transmission des données d'IEGM en temps réel sélectionnées tandis qu'une connexion de télémétrie entre le dispositif médical implantable (205) et l'appareil externe (250) est suspendue.

13. Le système décrit à la revendication 11, dans le cadre duquel le contrôleur est en outre conçu pour envoyer (320) depuis l'appareil externe (250) une instruction indiquant au dispositif médical implantable (205) de redémarrer la transmission des données d'IEGM en temps réel audit appareil externe (250) lorsqu'une saisie de la part de l'utilisateur est détectée après l'écoulement de la durée prédéterminée.

14. Le système décrit à la revendication 11, dans le cadre duquel l'instruction comprend une instruction indiquant d'éteindre un émetteur situé dans le dispositif médical implantable (205) et utilisé pour transmettre les données d'IEGM en temps réel.

15. Le système décrit à la revendication 11, dans le cadre duquel le contrôleur est en outre conçu pour demander périodiquement à l'utilisateur s'il souhaite continuer à recevoir les données d'IEGM en temps réel sur l'appareil externe (250).
